# EUROPEAN PATENT SPECIFICATION

(11) **EP 2 192 836 B1**
(45) Date of publication and mention of the grant of the patent: **18.04.2012**
(21) Application number: 08826714.1
(22) Date of filing: 28.07.2008
(51) Int. Cl.: A01N 43/56, A01P 7/04, A61K 31/4155

(54) **TREATMENT OF MYIASIS**
BEHANDLUNG VON MYISAIS
TRAITEMENT DE LA MYISASE

(30) Priority: 30.07.2007 US 962519 P
(43) Date of publication of application: 09.06.2010
(73) Proprietor: E. I. du Pont de Nemours and Company, Wilmington, DE 19898 (US)
(72) Inventor: TAYLOR, Wendy, Sue, Mount Laurel, New Jersey 08054 (US)
(74) Representative: Beacham, Annabel Rose
(86) International application number: PCT/US2008/071288
(87) International publication number: WO 2009/018186

(56) References cited:
- WO-A1-98/07423
- WO-A1-2004/067528
- WO-A1-2006/068669
- DATABASE CAB [Online] CAB INTERNATIONAL, WALLINGFORD, OXON, GB; 1997, P.N.R.Usherwood et al.: "Towards the development of ryanoid insecticides with low mammalian toxicity" XP002568747 retrieved from STN-International Database accession no. 97:25325 & TOXICOLOGY LETTERS, vol. 82/83, no. COM, 1995, pages 247-254,
- G.P.LAHM ET AL.: "Insecticidal anthranilic diamides: A new class of potent ryanodine receptor activators" BIOORGANIC & MEDICAL CHEMISTRY LETTERS, vol. 15, 13 September 2005 (2005-09-13), pages 4898-4906, XP002568748

## Description

### BACKGROUND OF THE INVENTION

Flies are not just a nuisance; they carry diseases which pose a serious health hazard to people and animals. Globally, they cause livestock and poultry production losses estimated in the billions of dollars. The growth and performance of nearly all farmed animals are adversely affected by flies, especially when they are present in high numbers. Infested animals become harassed and feed intake is drastically reduced. The result: significant reductions of meat, milk and egg production and serious economic losses.

Non-biting flies often feed on secretions from the eyes, nose and any small wounds of livestock This distracts animals from grazing, causing a reduction in growth and productivity. Non-biting flies are not key vectors of any specific disease organisms, but because of their feeding and reproduction habits, and the structure of their feet and mouthparts, they can act as mechanical vectors for a whole range of pathogens, from viruses to helminthes.

Biting flies can cause even greater irritation to domestic animals, and they too are vectors for disease transmission. However, because they feed on blood, they can also cause anemia and hypersensitivity. Biting flies therefore are considered by some to be a more serious problem in livestock production than non-biting flies.

However some non-biting flies often designated "blowflies" cause significant damage in their own right by virtue of their propensity to cause "myiesis" in susceptible animals. Myiasis is an animal or human disease caused by parasitic dipterous fly larvae feeding on the host's necrotic or living tissue. Blowflies are the single most important parasite of the sheep industry in Australia. Losses are estimated at more than $50 million yearly. These losses are caused by reduced growth of the sheep, reduced and inferior wool production, and extremely high labour costs expended in attempts to control the parasite.

Under normal conditions, blowflies do not attack live healthy sheep. If animals suffer open wounds, for example from branding or castration, some species of blowflies, deposit eggs in the wounds. These will hatch into maggots which eat the flesh of the animal.

The principal control method of adult populations of biting flies and blowflies involves topical insecticide applications to the livestock. Organophosphorus or organochlorine compounds are often used, usually in a spraying formulation.

There is a compelling need for improved insecticide formulations useful in the treatment of myiasis which the present invention addresses.

WO9807423 discloses the control of myiasis affecting cattle and sheep by Fipronil.

WO2006068669 discloses compositions comprising Cyantraniliprole in mixture with further pesticides and its use for the control of invertebrate pests.

### SUMMARY OF THE INVENTION

This invention relates to a composition comprising an parasiticidally effective amount of a compound of Formula 1, an *N*-oxide or a pharmaceutically or veterinarily acceptable salt thereof for use in treating myiasis of an animal

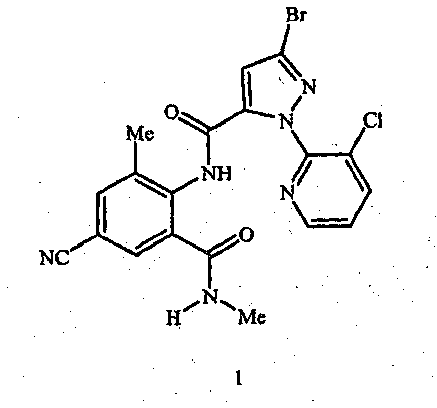

The compound of formula 1 is 3-bromo-1-(3-chloro-2-pyridinyl)-*N*-[4-cyano-2-methyl-6-[(methylamino)carbonyl]-phenyl]-1H-pyrazole-5-carboxamide.

The compositions of the present invention are those, which comprise the above compound. The methods of use are those involving the above-compound.

This invention also relates to a method of treating myiasis of an animal by applying to the animal a composition comprising a parasiticidally effective amount of the compound noted above.

The invention also comprises a compound of Formula 1 for use as a medicament.

The invention also relates to the use of a compound of Formula 1 in the manufacture of a medicament for the treatment of myiasis or the infestation of flies on an animal.

### DETAILS OF THE INVENTION

As used herein, the terms "comprises," "comprising," "includes," "including," "has," "having," "contains" or "containing," or any other variation thereof, are intended to cover a non-exclusive inclusion. For example, a composition, a mixture, process, method, article, or apparatus that comprises a list of elements is not necessarily limited to only those elements but may include other elements not expressly listed or inherent to such composition, mixture, process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

One skilled in the art will appreciate that not all nitrogen-containing heterocycles can form *N*-oxides since the nitrogen requires an available lone pair for oxidation to the oxide; one skilled in the art will recognize those nitrogen-containing heterocycles which can form *N*-oxides. One skilled in the art will also recognize that tertiary amines can form *N*-oxides. Synthetic methods for the preparation of *N*-oxides of heterocycles and tertiary amines are very well known by one skilled in the art including the oxidation of heterocycles and tertiary amines with peroxy acids such as peracetic and *m*-chloroperbenzoic acid (MCPBA), hydrogen peroxide, alkyl hydroperoxides such as *t*-butyl hydroperoxide, sodium perborate, and dioxiranes such as dimethydioxirane. These methods for the preparation of N-oxides have been extensively described and reviewed in the literature, see for example: T. L. Gilchrist in Comprehensive Organic Synthesis, vol. 7, pp 748-750, S. V. Ley, Ed., Pergamon Press; M. Tisler and B. Stanovnik in Comprehensive Heterocyclic Chemistry, vol. 3, pp 18-20, A. J. Boulton and A. McKillop, Eds., Pergamon Press; M. R. Grimmett and B. R. T. Keene in Advances in Heterocyclic Chemistry, vol. 43, pp 149-161, A. R. Katritzky, Ed., Academic Press; M. Tisler and B. Stanovnik in Advances in Heterocyclic Chemistry, vol. 9, pp 285-291, A. R. Katritzky and A. J. Boulton, Eds., Academic Press; and G. W. H. Cheeseman and E. S. G. Werstiuk in Advances in Heterocyclic Chemistry, vol. 22, pp 390-392, A. R. Katritzky and A. J. Boulton, Eds., Academic Press.

Compounds of this invention can exist as one or more stereoisomers. The various stereoisomers include enantiomers, diastereomers, atropisomers and geometric isomers. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers. Accordingly, the present invention comprises compounds selected from Formula 1, N-oxides and salts thereof. Pharmaceutically or veterinarily acceptable salts, suitable to the mode of administration, are contemplated. The compounds of the invention may be present as a mixture of stereoisomers, individual stereoisomers, or as an optically active form..

The salts of the compounds of the invention include acid-addition salts with inorganic or organic acids such as hydrobromic, hydrochloric, nitric, phosphoric, sulfuric, acetic, butyric, fumaric, lactic, maleic, malonic, oxalic, propionic, salicylic, tartaric, 4-toluenesulfonic or valeric acids. In the compositions and methods of this invention, the salts of the compounds of the invention are preferably acceptable for the veterinary/pharmaceutical uses described herein.

The compositions of the present invention are those, which comprise the above compounds. The methods of use are those involving the above-compounds.

"Flies" are insects in the order Diptera, meaning "two-winged". True flies have one pair of wings used for flying. Posterior to the wings is a pair of stalked knob-like structures (called halteres), which are organs of balance. Flies undergo complete metamorphosis, i.e. the life cycle consists of the following stages: egg, larva (called a maggot), pupa, and adult. Each stage of the life cycle may be a target for control and intervention.

Flies may be categorized into two functional categories "biting" and "non-biting".

"Biting flies" have specially adapted mouthparts well suited for piercing the host animal integument. The stable fly *Stomoxys calcitrans* is a good example of a biting fly. The stable fly has a proboscis which is used to pierce the skin and imbibe blood. Both the males and the females arc bloodkeders. The stable fly is often the only biting, blood-sucking fly breeding in any appreciable numbers in and around confined-animal production facilities. Another example of a biting fly is the horn fly, *Haematobia irritans irritans,* which like the stable fly is a bloodsucker and has great economic impact. Like the stable fly the horn fly has piercing/sucking mouthparts.

"Blowflies'" are defined as flies which are the etiologic agent of myiasis. By way of example the Calliphoridae family, together with the Sarcophagidae and the Oestridae families, contain the species largely responsible for many of the important myiases of domestic animals and man. Major species of blowflies include *Lucilia sericata* (greenbottles), *Phormia terraenovae* (blackbottles), *Calliphora erythrocephala* and *Calliphora. vomitoria* (bluebottles) in Europe. These flies are characterized by the color of the metallic sheen on their body sections. *Lucilia cuprina, L. caeser, L. illustris, Phormia regina, Calliphora stygia, C australis, C. fallax, Chrysomyia albiceps, C. chlorophyga, C. micropogon, and C. rufifacies* are examples of major species of blowflies in the tropics and subtropics. Blowflies are a particularly important problem in sheep farming.

The blowflies that attack sheep fall into two main categories:
(1) Primary flies, which are capable of initiating a strike on living sheep. These include Lucilia and Phormia spp. and some Calliphora spp.
(2) Secondary flies, which cannot initiate a strike, but attack an area already struck or otherwise damaged. They frequently extend the injury, rendering the strike one of great severity. Examples include many Calliphora spp. and, in warmer climates, Chrysomyia spp.

A "parasiticidally effective amount" is the amount of active ingredient needed to achieve an observable effect diminishing the occurrence or activity of the target invertebrate parasite pest. One skilled in the art will appreciate that the parasitically effective dose can vary for the various compounds and compositions of the present invention, the desired parasitical effect and duration, the target invertebrate pest species, the animal to be protected, the mode of application and the like, and the amount needed to achieve a particular result can be determined through simple experimentation

"Myiasis" is an animal disease caused by parasitic dipterous fly larvae feeding on the animal host's necrotic or living tissue. Colloquialisms for myiasis include "fly-strike" and "fly-blown". Blowfly myiasis is often associated with sheep; however, many other animals may be affected.

"Treating" or "Treatment" as it applies to myiasis or infestation refers to both the prevention and control of myiasis or infestation respectively

Embodiments of the present invention include:
**Embodiment 18.** The method of treatment of myiasis or use described in the Summary of the Invention wherein the myiasis is caused at least in part by larvae selected from the taxanomic families Calliphoridae, Sarcophagidae or Oestridae.
**Embodiment 19.** The method or use of Embodiment 18 wherein the myiasis is caused at least in part by larvae selected from the taxanomic families Calliphoridae, Sarcophagidae or Oestridae.
**Embodiment 20.** The method or use of Embodiment 18 wherein the myiasis is caused at least in part by larvae of the family Calliphoridae.
**Embodiment 21.** The method or use of Embodiment 18 wherein the myiasis is caused at least in part by larvae which are selected from the group consisting of *Lucilia cuprina* and *Lucilia sericata.*
**Embodiment 22.** The method or use Embodiment 18 wherein the myiasis is caused at least in part by larvae of *Lucilia cuprina.*
**Embodiment 23.** The method or use of Embodiment 18 wherein the myiasis is caused at least in part by larvae of *Lucilia sericata.*
**Embodiment 24.** The method or use Embodiment 18 wherein the animal is a cattle or sheep.
**Embodiment 25.** The method or use of any of Embodiments 18-24 wherein the composition comprises at least one additional component selected from the group consisting of solvents and/or carriers, emulsifiers and/or dispersing agents.
**Embodiment 26.** The method or use of Embodiment 25 and wherein the composition comprises at least one additional biologically active compound or agent.
**Embodiment 27.** The method or use of Embodiment 26 wherein the additional biologically active compound or agent is selected from the group consisting of macrocyclic lactones, acetyl cholinesterase inhibitors, arthropodgrowth regulators, GABA-gated chloride channel antagonists, mitochondrial electron transport inhibitors, nicotinic acetylcholine agonists/antagonists/activator, oxidative phosphorylation inhibitors, anthelminthics, sodium channel modulators or other antiparasitic compounds.
**Embodiment 28.** The method or use of Embodiment 27 wherein said biologically active compound is a macrocyclic lactone.
**Embodiment 29.** The method or use of Embodiment 27 wherein said biologically active compound is an acetyl cholinesterase inhibitor selected from the group of organophosphates and carbamates.
**Embodiment 30.** The method or use of Embodiment 27 wherein said biologically active compound is an arthropodgrowth regulator selected from the group of chitin synthesis inhibitors, ecdysone agonists/disruptors, lipid biosynthesis inhibitor and juvenile hormone mimics.
**Embodiment 31.** The method or use of Embodiment 27 wherein said biologically active compound is a GABA-gated chloride channel antagonist.
**Embodiment 32.** The method or use of Claim 27 wherein said biologically active compound is a mitochondrial electron transport inhibitor.
**Embodiment 33.** The method or use of Claim 27 wherein said biologically active compound is a nicotinic acetylcholine agonist/antagonist/activator.
**Embodiment 34.** The method or use of Claim 27 wherein said biologically active compound is an oxidative phosphorylation inhibitor.
**Embodiment 35.** The method or use of Claim 27 wherein said biologically active compound is an anthelminthic.

The embodiments above are intended to be illustrative and not limiting. Further aspects of the invention are discussed throughout the specification.

This invention also relates to a method of treating myiasis of an animal by applying to the animal a composition comprising a parasiticidally effective amount of a compound of Formula **1**, or an *N*-oxide, or a pharmaceutically or veterinarily acceptable salts salt thereof,

Therefore, the invention is understood to include the compounds described in the Summary of the Invention (and compositions containing them) for use as an anti-myiasis animal medicament.

The medicament may be presented in topical forms.

The invention is also understood to include the compounds described in the Summary of the Invention in the manufacture of medicaments for the protection of an animal myiasis.

The medicament may be presented in topical forms.

The invention is also understood to include the compounds described in the Summary of the Invention for use in the manufacture of medicaments for the protection of an animal from myiasis.

The medicament may be presented in topical forms.

The invention is also understood to include the compounds described in the Summary of the Invention packaged and presented for the protection of an animal myiasis.

The compounds of the invention may be packaged and presented as topical dosage forms.

The invention is also understood to include a process for manufacturing a composition for protecting an animal from the invertebrate parasitic pest characterized in that a compound of Formula 1 is admixed with at least one pharmaceutically or veterinarily acceptable carrier.

The compositions of the invention may be packaged and presented in topical dosage forms.

The compounds of Formula **1** which can be used according to the invention, have an excellent action against biting flies and blowflies, whilst being very well tolerated by animals. The invention thus represents a genuine enrichment of the art.

The compounds according to the invention possess a good ectoparasiticidal activity, whilst being of low toxicity to animals.

Since the compounds of Formula **1** are both effective adulticides, and larvacides i.e. since they are effective in both the adult stage of the target parasites, and the juvenile stages of the parasites they are particularly advantageous in the treatment of myiasis.

The compounds of Formula 1 can be prepared by the methods as described in US Patent Publication 2006/0111403A1 (herein incorporated by reference to the extent not inconsistent with the disclosure herein) and variations readily apparent to the skilled artisan. Synthetic methods for the preparation of *N*-oxides of heterocycles and tertiary amines are very well known by one skilled in the art including the oxidation of heterocycles and tertiary amines with peroxy acids such as peracetic and *m*-chloroperbenzoic acid (MCPBA), hydrogen peroxide, alkyl hydroperoxides such as *t*-butyl hydroperoxide, sodium perborate, and dioxiranes such as dimethydioxirane. These methods for the preparation of N-oxides have been extensively described and reviewed in the literature, see for example: T. L. Gilchrist in Comprehensive Organic Synthesis, vol. 7, pp 748-750, S. V. Ley, Ed., Pergamon Press; M. Tisler and B. Stanovnik in Comprehensive Heterocyclic Chemistry, vol. 3, pp 18-20, A. J. Boulton and A. McKillop, Eds., Pergamon Press; M. R. Grimmett and B. R. T. Keene in Advances in Heterocyclic Chemistry, vol. 43, pp 149-161, A. R. Katritzky, Ed., Academic Press; M. Tisler and B. Stanovnik in Advances in Heterocyclic Chemistry, vol. 9, pp 285-291, A. R. Katritzky and A. J. Boulton, Eds., Academic Press; and G. W. H. Cheeseman and E. S. G. Werstiuk in Advances in Heterocyclic Chemistry, vol. 22, pp 390-392, A. R. Katritzky and A. J. Boulton, Eds., Academic Press.

The following compound is expected to be advantageous in the practice of the invention.

**Table 1**

| |
|---|
| 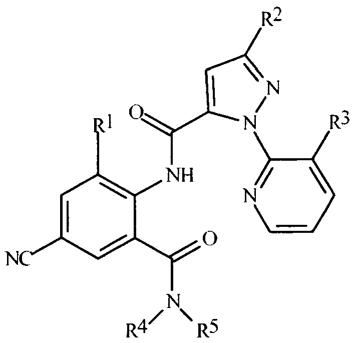 |

| R¹ | R² | R³ | R⁴ | R⁵ |
|---|---|---|---|---|
| | | | | |
| Me | Br | Cl | Me | H |

### Application of Compounds of the Invention

The compound of Formula 1 of this invention can be applied to any animal, including herd animals, that can be afflicted by myiasis. The compositions can be applied, for example, to cattle, sheep, goats, horses, donkeys, camels, pigs, reindeer, caribou and buffalo.

The "applying" can be accomplished by way of non limiting example, whole-animal sprays, self-applicating devices, pour on treatments and controlled-release devices, such as ear tags and tapes, neck collars, ear tags, tail bands, limb bands or halters which comprise compounds or compositions comprising compounds of Formula **1**. In addition to sprays and pour on treatments, application may be by other forms of topical administration, for example, in the form of immersion or dipping, washing, coating with powder, or application to a small area of the animal.

Application of the compositions according to the invention to the animals to be treated is done topically via solutions, emulsions, suspensions, (drenches), powders, and pour-on formulations.

The pour-on or spot-on method consists in applying the compound of Formula 1 to a specific location of the skin or coat, advantageously to the neck or backbone of the animal. This takes place e.g. by applying a swab or spray of the pour-on or spot-on formulation to a relatively small area of the coat, from where the active substance is dispersed almost automatically over wide areas of the fur owing to the spreading nature of the components in the formulation and assisted by the animal's movements.

The compounds of Formula **1** may be indirectly applied to an animal by applying it to the local environment in which the animal dwells (such as bedding, enclosures, or the like).

Whole-animal sprays provide rapid relief from fly pressure. Animal sprays are applied either as a dilute coarse spray, often applied under high pressure to soak the skin, or as a fine low-volume, more concentrated mist.

Self-applicating devices include back rubber covered with an absorbent material treated with an insecticide-oil solution, or dust bags filled with an insecticidal dust. Back rubbers and dustbags should be placed in gateways, near water and feed source, and in other areas where animals will make frequent contact with them.

Controlled-release ear tags and tapes are generally very effective for fly control in certain farm areas.

Pour-on treatments involves the application of an insecticide along the backline of the animal at a prescribed dosage of topical products. The pour-on or spot-on method is especially advantageous for use on herd animals such as cattle, horses, sheep or pigs, in which it is difficult or time-consuming to treat all the animals by more labor intensive methods of administration.

The compounds according to the invention are effective against fly larvae. The active compounds are employed in known manner, preferably by dermal or topical use, for example in the form of dipping, spraying, pour-on and spot-on, and powdering.

Blowfly strikes are almost always fatal unless the sheep is caught, the wool clipped from the infected area, the maggots scraped out, disinfectant or antibiotic and insecticide applied to prevent further strikes. Treatment of blowfly strike should aim to kill any maggots present, prevent the likelihood of further fly strike and assist the wound heal. The wool should be carefully clipped away from around the wound and surrounding area. A cream containing the compound of Formula **1** can be be applied to the infected areas. Mild cases should heal quickly with correct treatment.

It is also effective to treat myiasis afflicted sheep or those at risk of being afflicted by dipping. It is particularly important to immerse sheep for at least a full minute so as to ensure the dip saturates the whole fleece and regular replenishment of dip baths is important to maintain the strength of dip concentrate. Pour-ons would also be an effective treatment

### Compositions of the Invention

The compounds of the invention may be applied alone but are typically formulated into a veterinary or pharmaceutical composition. The compounds arc prepared or formulated into compositions in a known manner, for example by extending the active compounds with solvents and/or carriers, if appropriate using emulsifiers and/or dispersing agents; if, for example, water is used as the diluent, organic solvents can, if appropriate, be used as auxiliary solvents.

Typically a composition used in the present invention comprises a mixture of a compound of Formula **1**, an *N*-oxide or a salt thereof, with one or more pharmaceutically or veterinarily acceptable carriers comprising excipients and auxiliaries selected with regard to their suitability for topical administration and in accordance with standard practice. In addition, a suitable carrier is selected on the basis of compatibility with the one or more active ingredients in the composition, including such considerations as stability relative to pH and moisture content. The typical application medium will be a composition for protecting an animal from an invertebrate parasitic pest comprising a parasitically effective amount of a compound of Formula **1** and at least one carrier.

Formulations for topical administration are typically in the form of a powder, cream, suspension, spray, emulsion, foam, paste, aerosol, ointment, salve or gel. More typically a topical formulation is a water-soluble solution, which can be in the form of a concentrate that is diluted before use. Parasiticidal compositions suitable for topical administration typically comprise a compound of the present invention and one or more topically suitable carriers. In applications of a parasiticidal composition topically to the exterior of an animal as a line or spot (i.e. "spot-on" treatment), the active ingredient migrates over the surface of the animal to cover most or all of its external surface area. As a result, the treated animal is particularly protected from invertebrate pests that feed off the epidermis of the animal such as ticks, fleas and lice. Therefore formulations for topical localized administration often comprise at least one organic solvent to facilitate transport of the active ingredient over the skin and/or penetration into the epidermis of the animal. Carriers in such formulations include propylene glycol, paraffins, aromatics, esters such as isopropyl myristate, glycol ethers, alcohols such as ethanol, *n*-propanol, 2-octyl dodecanol or oleyl alcohol; solutions in esters of monocarboxylic acids, such as isopropyl myristate, isopropyl palmitate, lauric acid oxalic ester, oleic acid oleyl ester, oleic acid decyl ester, hexyl laurate, oleyl oleate, decyl oleate, caproic acid esters of saturated fatty alcohols of chain length C₁₂-C₁₈; solutions of esters of dicarboxylic acids, such as dibutyl phthalate, diisopropyl isophthalate, adipic acid diisopropyl ester, di-n-butyl adipate or solutions of esters of aliphatic acids, e.g., glycols. It may be advantageous for a crystallization inhibitor or a dispersant known from the pharmaceutical or cosmetic industry also to be present.

A pour-on formulation may also be prepared for control of parasites in an animal of agricultural worth. The pour-on formulations of this invention can be in the form of a liquid, powder, emulsion, foam, paste, aerosol, ointment, salve or gel. Typically, the pour-on formulation is liquid. These pour-on formulations can be effectively applied to sheep, cattle, goats, other ruminants, camelids, pigs and horses. The pour-on formulation is typically applied by pouring in one or several lines or in a spot-on the dorsal midline (back) or shoulder of an animal. More typically, the formulation is applied by pouring it along the back of the animal, following the spine. The formulation can also be applied to the animal by other conventional methods, including wiping an impregnated material over at least a small area of the animal, or applying it using a commercially available applicator, by means of a syringe, by spraying or by using a spray race. The pour-on formulations include a carrier and can also include one or more additional ingredients. Examples of suitable additional ingredients are stabilizers such as antioxidants, spreading agents, preservatives, adhesion promoters, active solubilisers such as oleic acid, viscosity modifiers, UV blockers or absorbers, and colourants. Surface active agents, including anionic, cationic, non-ionic and ampholytic surface active agents, can also be included in these formulations.

The formulations of this invention often include an antioxidant, such as BHT (butylated hydroxytoluene). The antioxidant is generally present in amounts of at 0.1-5% (wt/vol). Some of the formulations require a solubilizer, such as oleic acid, to dissolve the active agent. Common spreading agents used in these pour-on formulations are: isopropyl myristate (IPM), isopropyl palmitate (IPP), caprylic/capric acid esters of saturated C₁₂-C₁₈ fatty alcohols, oleic acid, oleyl ester, ethyl oleate, triglycerides, silicone oils and dipropylene glycol monomethyl ether (DPM). The pour-on formulations of this invention are prepared according to known techniques. Where the pour-on is a solution, the parasiticide/insecticide is mixed with the carrier or vehicle, using heat and stirring where required. Auxiliary or additional ingredients can be added to the mixture of active agent and carrier, or they can be mixed with the active agent prior to the addition of the carrier. If the pour-on is an emulsion or suspension, these formulations are similarly prepared using known techniques.

Other delivery systems for relatively hydrophobic pharmaceutical compounds may be employed. Liposomes and emulsions are well-known examples of delivery vehicles or carriers for hydrophobic drugs. In addition, organic solvents such as dimethylsulfoxide may be used.

The compounds of Formula **1** are generally present in the compositions in concentrations of 0.1 to 95 percent by weight, preferably 0.5 to 90 percent by weight. Preparations which are intended for direct application contain the active compound according to the invention in concentrations of between 0.001 and 5 percent by weight, preferably 0.005 to 3 percent by weight.

Dosages may range from 0.0001 mg/kg of animal body weight to about 1000 mg/kg. of the compound of Formula **1**. Sometimes dosages may be from 0.1 mg/kg of animal body weight to about 200 mg/kg. Often times it would be advantageous to administer amounts of about 0.01 to about 100 mg or between 0.02 to about 50mg/kg. and frequently between 0.1 and 75 mg/kg. Preferably, the treatment is carried out so as to administer to the animal a dose of from 0.1 to 40 mg/kgand in particular from 1 to 30 mg/kg. Administration may be given as a single dose or intermittent in time and may be administered daily, weekly, monthly, bimonthly or quarterly in order to achieve effective results in order to achieve effective results.

Nevertheless it can at times be necessary to deviate from the amounts mentioned, and in particular to do so in accordance with the body weight of the test animal and/or the method of application, but also because of the species of animal and its individual behavior towards the medicament, or the nature of the formulation of the latter and the time or interval at which it is administered. Thus it can suffice in some cases to manage with less than the above mentioned minimum amount while in other cases the upper limit mentioned must be exceeded. Where substantial amounts are applied, it can be advisable to divide these into several individual administrations over the course of the day. The general sense of the other statements made above also applies.

Pour-on or spot-on formulations suitably contain carriers, which promote rapid dispersement over the skin surface or in the coat of the host animal, and are generally regarded as spreading oils. Suitable carriers are e.g. oily solutions; alcoholic and isopropanolic solutions such as solutions of 2-octyldodecanol or oleyl alcohol; solutions in esters of monocarboxylic acids, such as isopropyl myristate, isopropyl palmitate, lauric acid oxalate, oleic acid oleyl ester, oleic acid decyl ester, hexyl laurate, oleyl oleate, decyl oleate, capric acid esters of saturated fat alcohols of chain length C.sub.12-C.sub.18; solutions of esters of dicarboxylic acids, such as dibutyl phthalate, diisopropyl isophthalate, adipic acid diisopropyl ester, di-n-butyl adipate or also solutions of esters of aliphatic acids, e.g. glycols. It may be advantageous for a dispersing agent to be additionally present, such as one known from the pharmaceutical or cosmetic industry. Examples are 2-pyrrolidone, 2-(N-alkyl)pyrrolidone, acetone, polyethylene glycol and the ethers and esters thereof, propylene glycol or synthetic triglycerides.

The oily solutions include e.g. vegetable oils such as olive oil, groundnut oil, sesame oil, pine oil, linseed oil or castor oil. The vegetable oils may also be present in epoxidised form. Paraffins and silicone oils may also be used.

A pour-on or spot-on formulation generally contains 1 to 20% by weight of a compound of Formula **1**, 0.1 to 50% by weight of dispersing agent and 45 to 98.9% by weight of solvent.

Importantly the compounds of Formula 1 may be indirectly applied to an animal by applying it to the local environment in which the animal dwells (such as bedding, enclosures, or the like). Effective use rates will range from about 1.0 to 50 mg/square meter but as little as 0.1 mg/square meter may be sufficient or as much as 150 mg/square meter may be required. One skilled in the art can easily determine the biologically effective amount necessary for the desired level of pest control.

### The Compositions of the Invention may comprise Additional Active Compounds:

It is contemplated that additional biologically active compounds may be be administered at the same time or separately over time to obtain broader spectrum of pest control or to attack adult fleas. Such additional biologically active compounds may be packaged together with the compound of Formula 1 as a kit. For convenience sake such additional biologically active compounds may be formulated into the same composition containing the compound of Formula **1**. Therefore the present invention contemplates the use of compositions characterised in that they contain, in addition to a compound of Formula **1**, further auxiliaries and/or active compounds, such as additional biologically active compounds, disinfectants or antibiotics may be admixed to the formulations, or the ready-to-use solutions, in addition to the customary solid or liquid extenders, diluents and/or surface-active agents.

Of note are additional biologically active compounds or agents selected from art-known anthelmintics, such as, for example, avermectins (e.g. ivermectin, moxidectin, milbemycin), benzimidazoles (e.g. albendazole, triclabendazole), salicylanilides (e.g. closantel, oxyclozanide), substituted phenols (e.g. nitroxynil), pyrimidines (e.g. pyrantel), imidazothiazoles (e.g. levamisole) and praziquantel.

Other biologically active compounds or agents useful in the compositions of the present invention can be selected from Insect Growth Regulators (IGRs) and Juvenile Hormone Analogues (JHAs) such as diflubenzuron, triflumuron, fluazuron, cyromazine, methoprene, etc., thereby providing both initial and sustained control of parasites (at all stages of insect development, including eggs) on the animal subject, as well as within the environment of the animal subject.

The compounds of Formula I according to the invention may be used alone or in combination with other biocides. They may be combined with pesticides having the same sphere of activity e.g. to increase activity, or with substances having another sphere of activity e.g. to broaden the range of activity. It can also be sensible to add so-called repellents. If the range of activity is to be extended to endoparasites, e.g. wormers, the compounds of Formula 1 are suitably combined with substances having endoparasitic properties. Of course, they can also be used in combination with antibacterial compositions.

Preferred groups of combination partners and especially preferred combination partners are named in the following, whereby combinations may contain one or more of these partners in addition to a compound of Formula 1.

Suitable partners in the mixture may be biocides, e.g. the insecticides and acaricides with a varying mechanism of activity, which are named in the following and have been known to the person skilled in the art for a long time, e.g. chitin synthesis inhibitors, growth regulators; active ingredients which act as juvenile hormones; active ingredients which act as adulticides; broad-band insecticides, broad-band acaricides and nematicides; and also the well known anthelminthics and insect- and/or acarid-deterring substances, and also repellents or detachers.

Examples of such biologically active compounds include but are not restricted to the following: Organophosphates, a class which are generally know to be inhibitors of acetyl cholinesterase: acephate, azamethiphos, azinphos-ethyl, azinphos-methyl, bromophos, bromophos-ethyl, cadusafos, chlorethoxyphos, chlorpyrifos, chlorfenvinphos, chlormephos, demeton, demeton-S-methyl, demeton-S-methyl sulphone, dialifos, diazinon, dichlorvos, dicrotophos, dimethoate, disulfoton, ethion, ethoprophos, etrimfos, famphur, fenamiphos, fenitrothion, fensulfothion, fenthion, flupyrazofos, fonofos, formothion, fosthiazate, heptenophos, isazophos, isothioate, isoxathion, malathion, methacriphos, methamidophos, methidathion, methyl-parathion, mevinphos, monocrotophos, naled, omethoate, oxydemeton-methyl, paraoxon, parathion, parathion-methyl, phenthoate, phosalone, phosfolan, phosphocarb, phosmet, phosphamidon, phorate, phoxim, pirimiphos, pirimiphos-methyl, profenofos, propaphos, proetamphos, prothiofos, pyraclofos, pyridapenthion, quinalphos, sulprophos, temephos, terbufos, tebupirimfos, tetrachlorvinphos, thimeton, triazophos, trichlorfon, vamidothion.

Carbamates, a class which are generally known to be inhibitors of acetyl cholinesterase: alanycarb, aldicarb, 2-sec-butylphenyl methylcarbamate, benfuracarb, carbaryl, carbofuran, carbosulfan, cloethocarb, ethiofencarb, fenoxycarb, fenthiocarb, furathiocarb, HCN-801, isoprocarb, indoxacarb, methiocarb, methomyl, 5 methyl-m-cumenylbutyryl(methyl) carbamate, oxamyi, pirimicarb, propoxur, thiodicarb, thiofanox, triazamate, UC-51717 Pyrethroids, a class which are generally known to be modulators of sodium channels: acrinathin, allethrin, alphametrin, 5-benzyl-3-furylmethyl (E)-(1 R)-cis-2,2-dimethyl-3-(2-oxothiolan-3-ylidenemethyl) cyclopropanecarboxylate, bifenthrin, ,8 cyfluthrin, cyfluthrin, oc-cypermethrin, ,8-cypermethrin, bioallethrin, bioallethrin((S)-I cyclopentylisomer), bioresmethrin, bifenthrin, NCI-85193, cycloprothrin, cyhalothrin, cythithrin, cyphenothrin, deltamethrin, empenthrin, esfenvalerate, ethofenprox, fenfluthrin, fenpropathrin, fenvalerate, flucythrinate, flumethrin, fluvalinate (D isomer), imiprothrin, cyhalothrin, \-cyhalothrin, permethrin, phenothrin, prallethrin, pyrethrins (natural products), resmethrin, tetramethrin, transfluthrin, theta-cypermethrin, silafluofen, T-fluvalinate, tefluthrin, tralomethrin, Zeta-cypermethrin.

Arthropod growth regulators including: a) chitin synthesis inhibitors: benzoylureas: chlorfluazuron, diflubenzuron, fluazuron, flucycloxuron, flufenoxuron, hexaflumuron, lufenuron, novaluron, teflubenzuron, triflumuron, buprofezin, diofenolan, hexythiazox, etoxazole, chlorfentazine; b) ecdysone agonists/disruptors: halofenozide, methoxyfenozide, tebufenozide; c) juvenoid hormone mimcs: pyriproxyfen, methoprene, fenoxycarb; d) lipid biosynthesis inhibitors: spirodiclofen. Other antiparasitics: acequinocyl, amitraz, AKD-1022, ANS-118, azadirachtin, Bacillus thuringiensis, bensultap, bifenazate, binapacryl, bromopropylate, BTG-504, I BTG-505, camphechlor, cartap, chlorobenzilate, chlordimeform, chlorfenapyr, chromafenozide, clothianidine, cyromazine, diacloden, diafenthiuron, DBI-3204, dinactin, dihydroxymethyidihydroxypyrrolidine, dinobuton, dinocap, endosulfan, ethiprole, ethofenprox, fenazaquin, flumite, MTI-800, fenpyroximate, fluacrypyrim, flubenzimine, flubrocythrinate, flufenzine, flufenprox, fluproxyfen, halofenprox, hydramethyinon, IKI-220, kanemite, NC-196, nccm guard, nidinorterfuran, nitenpyram, SD-35651, WL-108477, pirydaryl, propargite, protrifenbute, pymethrozine, pyridaben, pyrimidifen, NC-1111, R-195, RH-0345, RH-2485, RYI-210, S-1283, S-1833, SI-8601, silafluofen, silomadine, spinosad, tebufenpyrad, tetradifon, tetranactin, thiacloprid, thiocyclam, thiamethoxam, tolfenpyrad, triazamate, triethoxyspinosyn, trinactin, verbutin, vertalec, Y1-5301 Fungicides: acibenzolar, aldimorph, ampropylfos, andoprim, azaconazole, azoxystrobin, benalaxyl, benomyl, bialaphos, blasticidin-S, Bordeaux mixture, bromuconazole, bupirimate, carpropamid, captafol, captan, carbendazim, chlorfenazole, chloroneb, chloropicrin, chlorothalonil, chlozolinate, copper oxychloride, copper salts, cyflufenamid, cymoxanil, cyproconazole, cyprodinil, cyprofuram, RH-7281, diclocymet, diclobutrazole, diclomezine, dicloran, difenoconazole, RP-407213, dimethomorph, domoxystrobin, diniconazole, diniconazole-M, dodine, edifenphos, epoxiconazole, famoxadone, fenamidone, fenarimol, fenbuconazole, fencaramid, fenpiclonil, fenpropidin, fenpropimorph, fentin acetate, fluazinam, fludioxonil, flumetover, flumorf/flumorlin, fentin hydroxide, fluoxastrobin, fluquinconazole, flusilazole, flutolanil, flutriafol, folpet, fosetyl-aluminium, furalaxyl, furametapyr, hexaconazole, ipconazole, iprobenfos, iprodione, isoprothiolane, kasugamycin, krsoxim-methyl, mancozeb, maneb, mefenoxam, mepronil, metalaxyl, metconazole, metominostrobin/fenominostrobin, metrafenone, myclobutanil, neo-asozin, nicobifen, orysastrobin, oxadixyl, penconazole, pencycuron, probenazole, prochloraz, propamocarb, propioconazole, proquinazid, prothioconazole, pyrifenox, pyraclostrobin, pyrimethanil, pyroquilon, quinoxyfen, spiroxamine, sulfur, tebuconazole, tetreonazole, thiabendazole, thifluzamide, thiophanate-methyl, thiram, tiadinil, triadimefon, triadimenol, tricyclazole, trifioxystrobin, triticonazole, validamycin, vinclozin Biological agents: Bacillus thuringiensis ssp alzawai, kurstaki, Bacillus thuringiensis delta endotoxin, baculovirus, entomopathogenic bacteria, virus and fungi Bactericides: chlortetracycline, oxytetracycline, streptomycin,

Additional more specific examples of partner insecticides and acaricides are listed below:

| **Compound** | **Class** |
|---|---|
| Compound | Class |
| Abamectin | macrocyclic lactones |
| AC 303 630 | energy production modulator |
| Acephate | acetyl cholinesterase inhibitor |
| Acrinathrin | sodium channel modulator |
| Alanycarb | acetyl cholinesterase inhibitor |
| Aldicarb | acetyl cholinesterase inhibitor |
| alpha.-Cypermethrin | sodium channel modulator |
| Alphamethrin | sodium channel modulator |
| Amitraz | octopamine receptor ligand |
| Avermectin | macrocyclic lactones |
| Azinphos A | acetyl cholinesterase inhibitor |
| Azinphos M | acetyl cholinesterase inhibitor |
| Azinphos-methyl | acetyl cholinesterase inhibitor |
| Azocyclotin | oxidative phosphorylation inhibitor |
| Bacillus subtil. toxin | |
| Bendiocarb | acetyl cholinesterase inhibitor |
| Benfuracarb | acetyl cholinesterase inhibitor |
| Bensultap | nicotinic acetylcholine agonist/antagonist |
| beta.-Cyfluthrin | sodium channel modulator |
| Bifenthrin | sodium channel modulator |
| Brofenprox | sodium channel modulator |
| Bromophos A | acetyl cholinesterase inhibitor |
| Bufcncarb | acetyl cholinesterase inhibitor |
| Buprofezin | chitin synthesis inhibitor |
| Butocarboxin | acetyl cholinesterase inhibitor |
| Cadusafos | acetyl cholinesterase inhibitor |
| Carbaryl | acetyl cholinesterase inhibitor |
| Carbofuran | acetyl cholinesterase inhibitor |
| Carbophenthion | acetyl cholinesterase inhibitor |
| Cartap | nicotinic acetylcholine agonist/antagonist |
| Chloethocarb | acetyl cholinesterase inhibitor |
| Chlorethoxyfos | acetyl cholinesterase inhibitor |
| Chlorfenapyr | oxidative phosphorylation inhibitor |
| Chlorfluazuron | chitin synthesis inhibitor |
| Chlormephos | acetyl cholinesterase inhibitor |
| Chlorpyrifos | acetyl cholinesterase inhibitor |
| Cis-Resmethrin | sodium channel modulator |
| Clofentezine | |
| Cyanophos | acetyl cholinesterase inhibitor |
| Cycloprothrin | sodium channel modulator |
| Cyfluthrin | sodium channel modulator |
| Cyhexatin | oxidative phosphorylation inhibitor |
| D 2341 (bifenazate) | |
| Deltamethrin | sodium channel modulator |
| Demeton M | acetyl cholinesterase inhibitor |
| Demeton S | acetyl cholinesterase inhibitor |
| Demeton-S-methyl | acetyl cholinesterase inhibitor |
| Dichlofenthion | acetyl cholinesterase inhibitor |
| Dicliphos | acetyl cholinesterase inhibitor |
| Diethion | acetyl cholinesterase inhibitor |
| Diflubenzuron | chitin synthesis inhibitor |
| Dimethoate | acetyl cholinesterase inhibitor |
| Dimethylvinphos | acetyl cholinesterase inhibitor |
| Dioxathion | acetyl cholinesterase inhibitor |
| Doramectin | macrocyclic lactones |
| DPX-MP062 (indoxacarb) | sodium channel modulator |
| Edifenphos | acetyl cholinesterase inhibitor |
| Emamcctin | macrocyclic lactones |
| Endosulfan | gaba-gated chloride channel antagonist |
| Eprinomectin | macrocyclic lactones |
| Esfenvalerate | sodium channel modulator |
| Ethiofencarb | acetyl cholinesterase inhibitor |
| Ethion | acetyl cholinesterase inhibitor |
| Ethofenprox | sodium channel modulator |
| Ethoprophos | acetyl cholinesterase inhibitor |
| Etrimphos | acetyl cholinesterase inhibitor |
| Fenamiphos | acetyl cholinesterase inhibitor |
| Fenazacquin | mitochondrial electron transport inhibitor |
| Fenbutatin oxide | oxidative phosphorylation inhibitor |
| Fenitrothion | acetyl cholinesterase inhibitor |
| Fenobucarb (BPMC) | acetyl cholinesterase inhibitor |
| Fenothiocarb | acetyl cholinesterase inhibitor |
| Fenoxycarb | juvenile hormone mimic |
| Fenpropathrin | sodium channel modulator |
| Fenpyrad | mitochondrial electron transport inhibitor |
| Fenpyroximate | mitochondrial electron transport inhibitor |
| Fenthion | acetyl cholinesterase inhibitor |
| Fenvalerate | sodium channel modulator |
| Fipronil | gaba-gated chloride channel antagonist |
| Fluazinam | oxidative phosphorylation uncoupler |
| Fluazuron | chitin synthesis inhibitor |
| Flucycloxuron | chitin synthesis inhibitor |
| Flucythrinate | sodium channel modulator |
| Flufenoxuron | chitin synthesis inhibitor |
| Flufenprox | sodium channel modulator |
| Fonophos | acetyl cholinesterase inhibitor |
| Formothion | acetyl cholinesterase inhibitor |
| Fosthiazate | acetyl cholinesterase inhibitor |
| HCH | gaba-gated chloride channel antagonist |
| Heptenophos | acetyl cholinesterase inhibitor |
| Hexaflumuron | chitin synthesis inhibitor |
| Hexythiazox | |
| Hydroprene | juvenile hormone mimic |
| Imidacloprid | nicotinic acetylcholine agonist/antagonist |
| insect-active fungi | |
| insect-active nematodes | |
| insect-active viruses | |
| Iprobenfos | acetyl cholinesterase inhibitor |
| Isofenphos | acetyl cholinesterase inhibitor |
| Isoprocarb | acetyl cholinesterase inhibitor |
| Isoxathion | acetyl cholinesterase inhibitor |
| Ivermectin | chloride channel activator |
| lambda.-Cyhalothrin | sodium channel modulator |
| Lufenuron | chitin synthesis inhibitor |
| Malathion | acetyl cholinesterase inhibitor |
| Mecarbam | acetyl cholinesterase inhibitor |
| Mesulfenphos | acetyl cholinesterase inhibitor |
| Metaldehyd | |
| Methamidophos | acetyl cholinesterase inhibitor |
| Methiocarb | acetyl cholinesterase inhibitor |
| Methomyl | acetyl cholinesterase inhibitor |
| Methoprene | juvenile hormone mimic |
| Metolcarb | acetyl cholinesterase inhibitor |
| Mevinphos | acetyl cholinesterase inhibitor |
| Milbemectin | macrocyclic lactones |
| Moxidectin | macrocyclic lactones |
| Naled | acetyl cholinesterase inhibitor |
| NI-25, Acctamiprid | nicotinic acetylcholine agonist/antagonist |
| Nitenpyram | nicotinic acetylcholine agonist/antagonist |
| Nodulisporic acid/derivatives | macrocyclic lactones |
| Omethoat | acetyl cholinesterase inhibitor |
| Oxamyl | acetyl) cholinesterase inhibitor |
| Oxydemethon M | acetyl cholinesterase inhibitor |
| Oxydeprofos | acetyl cholinesterase inhibitor |
| Parathion | acetyl cholinesterase inhibitor |
| Parathion-methyl | acetyl cholinesterase inhibitor |
| Permethrin | sodium channel modulator |
| Phenthoate | acetyl cholinesterase inhibitor |
| Phorat | acetyl cholinesterase inhibitor |
| Phosalone | acetyl cholinesterase inhibitor |
| Phosmet | acetyl cholinesterase inhibitor |
| Phoxim | acetyl cholinesterase inhibitor |
| Pirimicarb | acetyl cholinesterase inhibitor |
| Pirimiphos A | acetyl cholinesterase inhibitor |
| Pirimiphos M | acetyl cholinesterase inhibitor |
| Promecarb | acetyl cholinesterase inhibitor |
| Propaphos | acetyl cholinesterase inhibitor |
| Propoxur | acetyl cholinesterase inhibitor |
| Prothiofos | acetyl cholinesterase inhibitor |
| Prothoat | acetyl cholinesterase inhibitor |
| Pyrachlophos | acetyl cholinesterase inhibitor |
| Pyradaphenthion | acetyl cholinesterase inhibitor |
| Pyresmethrin | sodium channel modulator |
| Pyrethrim | sodium channel modulator |
| Pyridaben | mitochondrial electron transport inhibitor |
| Pyrimidifen | mitochondrial electron transport inhibitor |
| Pyriproxyfen | juvenile hormone mimic |
| RH 5992 | ecdysone agonist |
| RH-2485 | ecdysone agonist |
| Salithion | acetyl cholinesterase inhibitor |
| selamectin | macrocyclic lactones |
| Silafluofen | sodium channel modulator |
| Spinosad | nicotinic acetylcholine activator |
| Sulfotep | acetyl cholinesterase inhibitor |
| Sulprofos | acetyl cholinesterase inhibitor |
| Tebufenozide | ecdysone agonist |
| Tebufenpyrad | mitochondrial electron transport inhibitor |
| Tebupirimphos | acetyl cholinesterase inhibitor |
| Teflubenzuron | chitin synthesis inhibitor |
| Tefluthrin | sodium channel modulator |
| Temephos | acetyl cholinesterase inhibitor |
| Tcrbufos | acetyl cholinesterase inhibitor |
| Tetrachlorvinphos | acetyl cholinesterase inhibitor |
| Thiafenox | |
| Thiodicarb | acetyl cholinesterase inhibitor |
| Thiofanox | acetyl cholinesterase inhibitor |
| Thionazin | acetyl cholinesterase inhibitor |
| Thuringiensin | |
| Tralomethrin | sodium channel modulator |
| Triarathen | |
| Triazamate | acetyl cholinesterase inhibitor |
| Triazophos | acetyl cholinesterase inhibitor |
| Trichlorfon | acetyl cholinesterase inhibitor |
| Triflumuron | chitin synthesis inhibitor |
| Trimethacarb | acetyl cholinesterase inhibitor |
| Vamidothion | acetyl cholinesterase inhibitor |
| XMC (3,5,-Xylyl-methylcarbamate) | acetyl cholinesterase inhibitor |
| Xylylcarb | acetyl cholinesterase inhibitor |
| YI 5301/5302 | |
| zeta.-Cypermethrin | sodium channel modulator |
| Zetamethrin | sodium channel modulator |

Non-limitative examples of suitable anthelminthics are named in the following, a few representatives have insecticidal and acaricidal activity in addition to the anthelminthic activity, and are partly already in the above list.
(A1) Praziquantel=2-cyclohexylcarbonyl-4-oxo-1,2,3,6,7,11b-hexahydr-o-4H-pyrazino[2,1-.alpha.]isoquinoline
(A2) Closantel=3,5-diiodo-N-[5-chloro-2-methyl-4-(a-cyano-4-chlorob-enzyl)phenyl]-salicylamide
(A3) Triclabendazole=5-chloro-6-(2,3-dichlorophenoxy)-2-methylthio--1H-benzimidazole
(A4) Levamisol=L-(-)-2,3,5,6-tetrahydro-6-phenylimidazo[2,1b]thiazo- 1e
(A5) Mebendazole=(5-benzoyl-1H-benzimidazol-2-yl)carbaminic acid methylester
(A6) Omphalotin=a macrocyclic fermentation product of the fungus Omphalotus olearius described In WO 97/20857
(A7) Abamectin=avermectin B1
(A8) Ivermectin=22,23-dihydroavermectin B1
(A9) Moxidectin=5-O-demethyl-28-deoxy-25-(1,3-dimethyl-1-butenyl)-6-,28-epoxy-23-(methoxyimino)-milbemycin B
(A10) Doramectin=2S-cyclohexyl-5-O-demethyl-25-de(1-methylpropyl)-a-vermectin A1a
(A11) Milbemectin=mixture of milbemycin A3 and milbemycin A4
(A12) Milbemycinoxim=5-oxime of milbemectin
Non-limitative examples of suitable repellents and detachers are:
(R1) DEET (N,N-diethyl-m-toluamide)
(R2) KBR 3023 N-butyl-2-oxycarbonyl-(2-hydroxy)-piperidine
(R3) Cymiazole=N,-2,3-dihydro-3-methyl-1,3-thiazol-2-ylidene-2,4-xy-lidene

The aforementioned partners in the mixture are best known to specialists in this field. Most are described in various editions of the Pesticide Manual, The British Crop Protection Council, London, and others in the various editions of The Merck Index, Merck & Co., Inc., Rahway, N.J., USA or in patent literature. Therefore, the following listing is restricted to a few places where they may be found by way of example.
(1) 2-Methyl-2-(methylthio)propionaldehyde-O-methylcarbamoyloxime (Aldicarb), from The Pesticide Manual, 11.sup.th Ed. (1997), The British Crop Protection Council, London, page 26;
(II) S-(3,4-dihydro-4-oxobenzo[d]-[1,2,3]-triazin-3-ylmethyl)O,O-di- methyl-phosphorodithioate (Azinphos-methyl), from The Pesticide Manual, 11.sup.thEd. (1997), The British Crop Protection Council, London, page 67;
(III) Ethyl-N-[2,3-dihydro-2,2-dimethylbenzofuran-7-yloxycarbonyl-(-methyl)aminothio]-N-isopropyl-.beta.-alaninate (Benfuracarb), from The Pesticide Manual, 11.sup.thEd. (1997), The British Crop Protection Council, London, page 96;
(IV) 2-Methylbiphenyl-3-ylmethyl-(Z)-(1RS)-cis-3-(2-chloro-3,3,3-tr-ifluoroprop-1-enyl)-2,2-dimethylcyclopropanecarboxylate (Bifenthrin), from The Pesticide Manual, 11.sup.thEd. (1997), The British Crop Protection Council, London, page 118;
(V) 2-tert-butylimino-3-isopropyl-5-phenyl-1,3,5-thiadiazian-4-one (Buprofezin), from The Pesticide Manual, 11.sup.thEd. (1997), The British Crop Protection Council, London, page 157;
(VI) 2,3-Dihydro-2,2-dimethylbenzofuran-7-yl-methylcarbamate (Carbofuran), from The Pesticide Manual, 11.sup.thEd. (1997), The British Crop Protection Council, London, page 186;
(VII) 2,3-Dihydro-2,2-dimethylbenzofuran-7-yl-(dibutylaminothio)met-hylcarbamate (Carbosulfan), from The Pesticide Manual, 11.sup.thEd. (1997), The British Crop Protection Council, London, page 188;
(VIII) S,S'-(2-dimethylaminotrimethylene)-bis(thiocarbamate) (Cartap), from The Pesticide Manual, 11.sup.thEd. (1997), The British Crop Protection Council, London, page 193;
(IX) 1-[3,5-Dichloro-4-(3-chloro-5-trifluoromethyl-2-pyridyloxy)phe- nyl]-3-(2,6-difluorobenzoyl)-urea (Chlorfluazuron), from The Pesticide Manual, 11.sup.thEd. (1997), The British Crop Protection Council, London, page 213;
(X) O,O-diethyl-O-3,5,6-trichloro-2-pyridyl-phosphorothioate (Chlorpyrifos), from The Pesticide Manual, 11.sup.thEd. (1997), The British Crop Protection Council, London, page 235;
(XI) (RS)-.alpha.-cyano-4-fluoro-3-phenoxybenzyl-(1RS,3RS;1RS,3RS)-- 3-(2,2-dichlorovinyl)-2,2-di-methylcyclopropanecarboxylate (Cyfluthrin), from The Pesticide Manual, 11.sup.thEd. (1997), The British Crop Protection Council, London, page 293; (XII) Mixture of (S)-.alpha.-cyano-3-phenoxybenzyl-(Z)-(1R,3R)-3-(2- -chloro-3,3,3-trifluoropropenyl)-2,2-dimethylcyclopropanecarboxylate and (R)-.alpha.-cyano-3-phenoxybenzy)-(Z)-(1R,3)-3-(2-chloro-3,3,3-trifluorop-ropenyl)-2,2-dimethylcyclopropanecarboxylate (Lambda-Cyhalothrin), from The Pesticide Manual, 11.sup.thEd. (1997), The British Crop Protection Council, London, page 300;
(XIII) Racemate consisting of (S)-.alpha.-cyano-3-phenoxybenzyl-(2)- -(1R,3R)-3-(2,2-dichlorovinyl)-2,2-dimethylcyclopropanecarboxylate and (R)-.alpha.-cyano-3-phenoxybenzyl-(1S,3S)-3-(2,2-dichlorovinyl)-2,2-dimet-hylcyclopropanecarboxylate (Alpha-cypermethrin), from The Pesticide Manual, 11.sup.thEd. (1997), The British Crop Protection Council, London, page 308;
(XIV) a mixture of the stereoisomers of (S)-.alpha.-cyano-3-phenoxy- benzyl (1RS,3RS,1 RS,3RS)-3-(2,2-dichlorovinyl)-2,2-dimethylcyclopropaneca- rboxylate (zeta-Cypermethrin), from The Pesticide Manual, 11.sup.thEd. (1997), The British Crop Protection Council, London, page 314;
(XV) (S)-.alpha.-cyano-3-phenoxybenzyl-(1R,3R)-3-(2,2-dibromovinyl)- -2,2-dimethylcyclopropanecarboxylate (Deltamethrin), from The Pesticide Manual, 11.sup.thEd. (1997), The British Crop Protection Council, London, page 344;
(XVI) (4-chlorophenyl)-3-(2,6-difluorobenzoyl)urea (Diflubenzuron), from The Pesticide Manual, 11.sup.thEd. (1997), The British Crop Protection Council, London, page 395; (XVII) (1,4,5,6,7,7-Hexachloro-8,9,10-trinorborn-5-en-2,3-ylenebism- ethylene)-sulphite (Endosulfan), from The Pesticide Manual, 11.sup.thEd. (1997), The British Crop Protection Council, London, page 459;
(XVIII) .alpha.-ethylthio-o-tolyl-methylcarbamate (Ethiofencarb), from The Pesticide Manual, 11.sup.thEd. (1997), The British Crop Protection Council, London, page 479; (XIX) O,O-dimethyl-O-4-nitro-m-tolyl-phosphorothioate (Fenitrothion), from The Pesticide Manual, 11.sup.thEd. (1997), The British Crop Protection Council, London, page 514; (XX) 2-sec-butylphenyl-methylcarbamate (Fenobucarb), from The Pesticide Manual, 11.sup.thEd. (1997), The British Crop Protection Council, London, page 516;
(XXI) (RS)-.alpha.-cyano-3-phenoxybenzyl-(RS)-2-(4-chlorophenyl)-3-- methylbutyrate (Fenvalerate), from The Pesticide Manual, 11.sup.thEd. (1997), The British Crop Protection Council, London, page 539;
(XXII) S-[formyl(methyl)carbamoylmethyl]-O,O-dimethyl-phosphorodith- ioate (Formothion), from The Pesticide Manual, 11.sup.thEd. (1997), The British Crop Protection Council, London, page 625;
(XXIII) 4-Methylthio-3,5-xylyl-methylcarbamate (Methiocarb), from The Pesticide Manual, 11.sup.thEd. (1997), The British Crop Protection Council, London, page 813;
(XXIV) 7-Chlorobicyclo[3.2.0]hepta-2,6-dien-6-yl-dimethylphosphate (Heptenophos), from The Pesticide Manual, 11.sup.thEd. (1997), The British Crop Protection Council, London, page 670;
(XXV) 1-(6-chloro-3-pyridylmethyl)-N-nitroimidazolidin-2-ylidenamin- e (Imidacloprid), from The Pesticide Manual, 11.sup.thEd. (1997), The British Crop Protection Council, London, page 706;
(XXVI) 2-isopropylphenyl-methylcarbamate (isoprocarb), from The Pesticide Manual, 11.sup.thEd. (1997), The British Crop Protection Council, London, page 729;
(XXVII) O,S-dimethyl-phosphoramidothioate (Methamidophos), from The Pesticide Manual, 11.sup.thEd. (1997), The British Crop Protection Council, London, page 808; (XXVIII) S-Methyl-N-(methylcarbamoyloxy)thioacetimidate (Methomyl), from The Pesticide Manual, 11.sup.thEd. (1997), The British Crop Protection Council, London, page 815;
(XXIX) Methyl-3-(dimethoxyphosphinoyloxy)but-2-enoate (Mevinphos), from The Pesticide Manual, 11.sup.thEd. (1997), The British Crop Protection Council, London, page 844;
(XXX) O,O-diethyl-O-4-nitrophenyl-phosphorothloate (Parathion), from The Pesticide Manual, 11.sup.thEd. (1997), The British Crop Protection Council, London, page 926; (XXXI) O,O-dimethyl-O-4-nitrophenyl-phosphorothioate (Parathion-methyl), from The Pesticide Manual, 11.sup.thEd. (1997), The British Crop Protection Council, London, page 928;
(XXXII) S-6-chloro-2,3-dihydro-2-oxo-1,3-benzoxazol-3-ylmethyl-O,O-- diethyl-phosphordithioate (Phosalone), from The Pesticide Manual, 11.sup.thEd. (1997), The British Crop Protection Council, London, page 963;
(XXXIII) 2-Dimethylamino-5,6-dimethylpyrimidin-4-yl-dimethylcarbama- te (Pirimicarb), from The Pesticide Manual, 11.sup.thEd. (1997), The British Crop Protection Council, London, page 985;
(XXXIV) 2-isopropoxyphenyl-methylcarbamate (Propoxur), from The Pesticide Manual, 11.sup.thEd. (1997), The British Crop Protection Council, London, page 1036;
(XXXV) 1-(3,5-dichloro-2,4-difluorophenyl)-3-(2,6-difluorobenzoyl)u- rea (Teflubenzuron), from The Pesticide Manual, 11.sup.thEd. (1997), The British Crop Protection Council, London, page 1158;
(XXXVI) S-tert-butylthiomethyl-O,O-dimethyl-phosphorodithioate (Terbufos), from The Pesticide Manual, 11.sup.thEd. (1997), The British Crop Protection Council, London, page 1165;
(XXXVII) ethyl-(3-tert.-butyl-1-dimethylcarbamoyl-1H-1,2,4-triazol-- 5-yl-thio)-acetate, (Triazamate), from The Pesticide Manual, 11.sup.thEd. (1997), The British Crop Protection Council, London, page 1224;
(XXXVIII) Abamectin, from The Pesticide Manual, 11.sup.thEd. (1997), The British Crop Protection Council, London, page 3;
(XXXIX) 2-sec-butylphenyl-methylcarbamate (Fenobucarb), from The Pesticide Manual, 11.sup.thEd. (1997), The British Crop Protection Council, London, page 516;
(XL) N-tert.-butyl-N'-(4-ethylbenzoyl)-3,5-dimethylbenzohydrazide (Tebufenozide), from The Pesticide Manual, 11.sup.thEd. (1997), The British Crop Protection Council, London, page 1 147;
(XLI) (.+-.)-5-amino-1-(2,6-dichloro-.alpha.,.alpha.,.alpha.-triflu- oro-p-tolyl)-4-trifluoromethyl-sulphinylpyrazol-3-carbonitrile (Fipronil), from The Pesticide Manual, 11.sup.thEd. (1997), The British Crop Protection Council, London, page 545;
(XLII) (RS)-.alpha.-cyano-4-fluoro-3-phenoxybenzyl(1RS,3RS;1RS,3RS)-3-(2,2-dichlorovinyl)-2,2-dimethylcyclopropanecarboxylate (beta-Cyfluthrin), from The Pesticide Manual, 11.sup.thEd. (1997), The British Crop Protection Council, London, page 295; (XLIII) (4-ethoxyphenyl)-[3-(4-fluoro-3-phenoxyphenyl)propyl](dimet- hyl)silane (Silafluofen), from The Pesticide Manual, 11.sup.thEd. (1997), The British Crop Protection Council, London, page 1105;
(XLIV) tert.-butyl (E)-.alpha.-(1,3-dimethyl-5-phenoxypyrazol-4-yl-- methylenamino-oxy)-p-toluate (Fenpyroximate), from The Pesticide Manual, 11.sup.thEd. (1997), The British Crop Protection Council, London, page 530;
(XLV) 2-tert.-butyl-5-(4-tert.-butylbenzylthio)-4-chloropyridazin-3- (2H)-one (Pyridaben), from The Pesticide Manual, 11.sup.thEd. (1997), The British Crop Protection Council, London, page 1161;
(XLVI) 4-[[4-(1,1-dimethylphenyl)phenyl]ethoxy]-quinazoline (Fenazaquin), from The Pesticide Manual, 11.sup.thEd. (1997), The British Crop Protection Council, London, page 507;
(XLVII) 4-phenoxyphenyl-(RS)-2-(pyridyloxy)propyl-ether (Pyriproxyfen), from The Pesticide Manual, 11.sup.thEd. (1997), The British Crop Protection Council, London, page 1073;
(XLVIII) 5-chloro-N-{2-[4-(2-ethoxyethyl)-2,3-dimethylphenoxy]ethyl-}-6-ethylpyrimidine-4-amine (Pyrimidifen), from The Pesticide Manual, 11.sup.thEd. (1997), The British Crop Protection Council, London, page 1070;
(XLIX) (E)-N-(6-chloro-3-pyridylmethyl)-N-ethyl-N'-methyl-2-nitrovi- nylidenediamine (Nitenpyram), from The Pesticide Manual, 11.sup.thEd. (1997), The British Crop Protection Council, London, page 880;
(L) (E)-N.sup.1-[(6-chloro-3-pyridyl)methyl]-N.sup.2-cyano-N.sup.1-- methylacetamidine (NI-25, Acetamiprid), from The Pesticide Manual, 11.sup.thEd. (1997), The British Crop Protection Council, London, page 9;
(LI) Avermectin B.sub.1, from The Pesticide Manual, 11.sup.thEd. (1997), The British Crop Protection Council, London, page 3;
(LII) an insect-active extract from a plant, especially (2R,6aS,12aS)-1,2,6,6a,12,12a-hexhydro-2-isopropenyl-8,9-dimethoxy-chrome- no[3,4-b]furo[2,3-h]chromen-6-one (Rotenone), from The Pesticide Manual, 11.sup.thEd. (1997), The British Crop Protection Council, London, page 1097; and an extract from Azadirachta indica, especially azadirachtin, from The Pesticide Manual, 11.sup.thEd. (1997), The British Crop Protection Council, London, page 59; and
(LII) a preparation which contains insect-active nematodes, preferably Heterorhabditis bactedophora and Heterorhabditis megidis, from The Pesticide Manual, 11.sup.thEd. (1997), The British Crop Protection Council, London, page 671; Steinemema feltiae, from The Pesticide Manual, 11.sup.thEd. (1997), The British Crop Protection Council, London, page 1115 and Steinemema scaptedsci, from The Pesticide Manual, 11.sup.thEd. (1997), The British Crop Protection Council, London, page 1116;
(LIV) a preparation obtainable from Bacillus subtilis, from The Pesticide Manual, 11.sup.thEd. (1997), The British Crop Protection Council, London, page 72; or from a strain of Bacillus thuringiensis with the exception of compounds isolated from GC91 or from NCTC11821; The Pesticide Manual, 11.sup.thEd. (1997), The British Crop Protection Council, London, page 73;
(LV) a preparation which contains insect-active fungi, preferably Verticillium lecanii, from The Pesticide Manual, 11.sup.th Ed. (1997), The British Crop Protection Council, London, page 1266; Beauveda brogniartii, from The Pesticide Manual, 11.sup.thEd. (1997), The British Crop Protection Council, London, page 85 and Beauveda bassiana, from The Pesticide Manual, 11.sup.thEd. (1997), The British Crop Protection Council, London, page 83;
(LVI) a preparation which contains insect-active viruses, preferably Neodipridon Sertifer NPV, from The Pesticide Manual, 11.sup.thEd. (1997), The British Crop Protection Council, London, page 1342; Mamestra brassicae NPV, from The Pesticide Manual, 11.sup.thEd. (1997), The British Crop Protection Council, London, page 759 and Cydia pomonella granulosis virus, from The Pesticide Manual, 11.sup.thEd. (1997), The British Crop Protection Council, London, page 291;
(CLXXXI) 7-chloro-2,3,4a,5-tetrahydro-2-[methoxycarbonyl(4-trifluor- omethoxyphenyl)-carbamoyl]indol[1,2e]oxazoline-4a-carboxylate (DPX-MP062, Indoxycarb), from The Pesticide Manual, 11.sup.thEd. (1997), The British Crop Protection Council, London, page 453;
(CLXXXII) N'-tert.-butyl-N'-(3,5-dimethylbenzoyl)-3-methoxy-2-methy- lbenzohydrazide (RH-2485, Methoxyfenozide), from The Pesticide Manual, 11.sup.thEd. (1997), The British Crop Protection Council, London, page 1094; and
(CLXXXIII) (N'-[4-methoxy-biphenyl-3-yl]-hydrazinecarboxylic acid isopropylester (D 2341), from Brighton Crop Protection Conference, 1996, 487-493; (R2) Book of Abstracts, 212th ACS National Meeting Orlando, Fla., August 25-29 (1996), AGRO-020. Publisher: American Chemical Society, Washington, D.C. CONEN: 63BFAF.

As a rule, the anthelminthic compositions according to the invention contain 0.1 to 99% by weight, especially 0.1 to 95% by weight of active ingredient of Formula 1 mixtures thereof, 99.9 to 1% by weight, especially 99.8 to 5% by weight of a solid or liquid admixture, including 0 to 25% by weight, especially 0.1 to 25% by weight of a surfactant.

As noted above, in another embodiment of the process according to the invention, compounds of Formula 1 and the additional compounds noted hereinbefore may be applied in a distinct and separate manner over time.

The following TESTS demonstrate the control efficacy of compounds of this invention on specific pests. The pest control protection afforded by the compounds is not limited, however, to these species.

### BIOLOGICAL EXAMPLES OF THE INVENTION

### TEST A

Adult *L. serricata* (blowflies): comparative example, not forming part of the invention Adult flies are placed on beds of blood agar (in individual test wells; 4 flies per well) in which test compound (3-bromo-1-(3-chloro-2-pyridinyl)-*N*-[4-cyano-2-methyl-6-[(methylamino)carbonyl]phenyl]-1*H*-pyrazole-5-carboxamide)was dissolved/suspended prior to hardening of agar. Flies can take up test compound by both ingestion and contact. Assays are scored by number of dead flies at 2 hours, 4 hours, and 24 hours.

| | **Number flies in test** | **Number dead flies at 2 hours** | | **Number dead flies at 4 hours** | | **Number dead flies at 24 hours** | |
|---|---|---|---|---|---|---|---|
| **Rate (ppm)** | | **Chlorop yriphos** | **Test Compound** | **Chlorop yriphos** | **Test Compound** | **Chlorop yriphos** | **Test Compound** |
| **100** | 4 | 4 | 0 | 4 | 0 | 4 | 3 |
| **50** | 4 | 4 | 0 | 4 | 0 | 4 | 3 |
| **25** | 4 | 3 | 0 | 4 | 0 | 4 | 2 |
| **10** | 4 | 1 | 0 | 4 | 0 | 4 | 1 |
| **1** | 4 | 0 | 0 | 1 | 0 | 4 | 0 |
| **Untreated** | 16 | 0 | 0 | 0 | 0 | 0 | 0 |

### L. serricata (insect; blowfly) larvae.

Test compound (3-bromo-1-(3-chloro-2-pyridinyl)-*N*-[4-cyano-2-methyl-6-[(methylamino)-carbonyl]phenyl]-1*H*-pyrazole-5-carboxamide) was mixed with dried blood bovine serum and placed on filter paper discs. Newly emerged larvae of blowfly, Lucilica scricata were added to the filter paper, which were ingested by larvae. Activity may occur through both feeding and contact. Four replicates were run per data point. Activity was assessed at 24 hours.
Results: Test compound gave 90-100% mortality at 0.5ppm at 24 hours. Fipronil gave 90-100% mortality at 0.5ppm at 24 hours.

## Claims

1. A composition comprising an parasiticidally effective amount of a compound of Formula 1, an N-oxide or a pharmaceutically or veterinarily acceptable salt thereof for use in treating myiasis of an animal

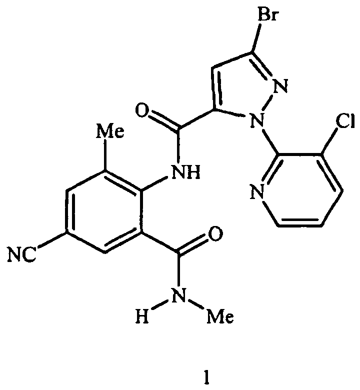

2. The composition of Claim 1 wherein the myiasis is caused at least in part by larvae selected from the taxanomic families Calliphoridae, Sarcophagidae or Oestridae.

3. The composition of Claim 1 wherein the myiasis is caused at least in part by larvae of the family Calliphoridae.

4. The composition of Claim 1 wherein the myiasis is caused at least in part by larvae which are selected from the group consisting of *Lucilia cuprina* and *Lucilia sericata.*

5. The composition of Claim 1 wherein the myiasis is caused at least in part by larvae of *Lucilia cuprina.*

6. The composition of Claim 1 wherein the myiasis is caused at least in part by larvae of *Lucilia sericata.*

7. The composition of Claim 1 for use in treating myiasis of an animal according to claim 1, wherein the animal is a cattle or sheep.

8. The composition of Claims 1-7 for use in treating myiasis of an animal according to claim 1, wherein the composition comprises at least one additional component selected from the group consisting of solvents and/or carriers, emulsifiers and/or dispersing agents.

9. The composition of Claim 8 for use in treating myiasis of an animal according to claim 1, wvherein the composition comprises at least one additional biologically active compound or agent.

10. The composition of Claim 9 for use in treating myiasis of an animal according to claim 1, wherein the additional biologically active compound or agent is selected from the group consisting of macrocyclic lactones, acetyl cholinesterase inhibitors, arthropodgrowth regulators, GABA-gated chloride channel antagonists, mitochondrial electron transport inhibitors, nicotinic acetylcholine agonists/antagonists/activator, oxidative phosphorylation inhibitors, anthelminthics, sodium channel modulators or other antiparasitic compounds.

11. The composition of Claim 10 for use in treating myiasis of an animal according to claim 1, wherein said biologically active compound is a macrocyclic lactone.

12. The composition of Claim 10 for use in treating myiasis of an animal according to claim 1, wherein said biologically active compound is an 7acetyl cholinesterase inhibitor selected from the group of organophosphates and carbamates.

13. The composition of Claim 10 for use in treating myiasis of an animal according to claim 1, wherein said biologically active compound is an arthropodgrowth regulator selected from the group of chitin synthesis inhibitors, ecdysone agonists/disruptors, lipid biosynthesis inhibitor and juvenile hormone mimics.

14. The composition of Claim 10 for use in treating myiasis of an animal according to claim 1, wherein said biologically active compound is a GABA-gated chloride channel antagonist.

15. The composition of Claim 10 for use in treating myiasis of an animal according to claim 1, wherein said biologically active compound is a mitochondrial electron transport inhibitor.

16. The composition of Claim 10 for use in treating myiasis of an animal according to claim 1, wherein said biologically active compound is a nicotinic acetylcholine agonist/antagonist/activator.

17. The composition of Claim 10 for use in treating myiasis of an animal according to claim 1, wherein said biologically active compound is an oxidative phosphorylation inhibitor.

18. The composition of Claim 10 for use in treating myiasis of an animal according to claim 1, wherein said biologically active compound is an anthelminthic.

19. The composition of Claim 10 for use in treating myiasis of an animal according to claim 1, wherein said biologically active compound is a sodium channel modulator.

## Patentansprüche

1. Zusammensetzung, die eine parasitizid wirksame Menge einer Verbindung gemäß Formel 1, ein N-Oxid oder ein pharmazeutisch oder tiermedizinisch akzeptierbares Salz davon zur Verwendung bei der Behandlung von Myiasis (Madenkrankheit) bei einem Tier aufweist,

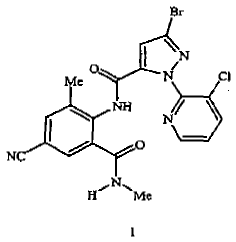

2. Zusammensetzung nach Anspruch 1, wobei die Myiasis zumindest teilweise durch Larven verursacht wird, die unter den taxonomischen Familien Calliphoridae, Sarcophagidae oder Oestridae ausgewählt sind.

3. Zusammensetzung nach Anspruch 1, wobei die Myiasis zumindest teilweise durch Larven der Familie Calliphoridae verursacht wird.

4. Zusammensetzung nach Anspruch 1, wobei die Myiasis zumindest teilweise durch Larven verursacht wird, die aus der Gruppe ausgewählt sind, die aus Lucilia cuprina und Lucilia sericata besteht.

5. Zusammensetzung nach Anspruch 1, wobei die Myiasis zumindest teilweise durch Larven von Lucilia cuprina verursacht wird.

6. Zusammensetzung nach Anspruch 1, wobei die Myiasis zumindest teilweise durch Larven von Lucilia sericata verursacht wird.

7. Zusammensetzung nach Anspruch 1 zur Verwendung bei der Behandlung von Myiasis bei einem Tier gemäß Anspruch 1, wobei das Tier ein Rind oder Schaf ist.

8. Zusammensetzung nach einem der Ansprüche 1-7 zur Verwendung bei der Behandlung von Myiasis bei einem Tier gemäß Anspruch 1, wobei die Zusammensetzung mindestens eine zusätzliche Komponente aufweist, die aus der Gruppe ausgewählt ist, die aus Lösungsmitteln und/oder Trägern Emulgatoren und/oder Dispergiermitteln besteht.

9. Zusammensetzung nach Anspruch 8 zur Verwendung bei der Behandlung von Myiasis bei einem Tier gemäß Anspruch 1, wobei die Zusammensetzung mindestens eine zusätzliche biologisch aktive Verbindung oder einen Wirkstoff aufweist.

10. Zusammensetzung nach Anspruch 9 zur Verwendung bei der Behandlung von Myiasis bei einem Tier gemäß Anspruch 1, wobei die zusätzliche biologisch aktive Verbindung oder der Wirkstoff aus der Gruppe ausgewählt ist, die aus makrocyclischen Lactonen, Acetylcholinesterase-Inhibitoren, Arthropoden-Wachstumsregulatoren, Antagonisten zum GABA-kontrollierten Chloridkanal Inhibitoren des mitochondrialen Elektronentransports, Agonisten/Antagonisten/Aktivator für Nicotinsäureacetylcholin, Inhibitoren der oxidativen Phosphorylierung, Anthelminthika, Natriumkanal-Modulatoren oder anderen antiparasitischen Verbindungen besteht.

11. Zusammensetzung nach Anspruch 10 zur Verwendung bei der Behandlung von Myiasis bei einem Tier gemäß Anspruch 1, wobei die biologisch aktive Verbindung ein makrocyclisches Lacton ist.

12. Zusammensetzung nach Anspruch 10 zur Verwendung bei der Behandlung von Myiasis bei einem Tier gemäß Anspruch 1, wobei die biologisch aktive Verbindung ein Acetylcholinesterase-Inhibitor ist, der aus der Gruppe ausgewählt ist, die aus Organophosphaten und carbamaten besteht.

13. Zusammensetzung nach Anspruch 10 zur Verwendung bei der Behandlung von Myiasis bei einem Tier gemäß Anspruch 1, wobei die biologisch aktive Verbindung ein Arthropoden-Wachstumsregulator ist, der aus der Gruppe ausgewählt ist, die aus Chitinsynthese-Inhibitoren, Ecdyson-Agonisten/Disruptoren, Lipidbiosynthese-Inhibitoren und Juvenilhormon-Imitatoren besteht.

14. Zusammensetzung nach Anspruch 10 zur Verwendung bei der Behandlung von Myiasis bei einem Tier gemäß Anspruch 1, wobei die biologisch aktive Verbindung ein Antagonist zu einem GABA-kontrollierten Chloridkanal ist.

15. Zusammensetzung nach Anspruch 10 zur Verwendung bei der Behandlung von Myiasis bei einem Tier gemäß Anspruch 1, wobei die biologisch aktive Verbindung ein Inhibitor des mitochondrialen Elektronentransports ist.

16. Zusammensetzung nach Anspruch 10 zur Verwendung bei der Behandlung von Myiasis bei einem Tier gemäß Anspruch 1, wobei die biologisch aktive Verbindung ein Agonist/Antagonist/Aktivator von Nicotinsäureacetylcholin ist.

17. Zusammensetzung nach Anspruch 10 zur Verwendung bei der Behandlung von Myiasis bei einem Tier gemäß Anspruch 1, wobei die biologisch aktive Verbindung ein Inhibitor der oxidativen Phosphorylierung ist.

18. Zusammensetzung nach Anspruch 10 zur Verwendung bei der Behandlung von Myiasis bei einem Tier gemäß Anspruch 1, wobei die biologisch aktive Verbindung ein Anthelminthikum ist.

19. Zusammensetzung nach Anspruch 10 zur Verwendung bei der Behandlung von Myiasis bei einem Tier gemäß Anspruch 1, wobei die biologisch aktive Verbindung ein Natriumkanal-Modulator ist.

## Revendications

1. Composition comprenant une quantité efficace du point de vue parasiticide d'un composé de la Formule I, d'un N-oxyde ou d'un sel pharmaceutiquement ou vétérinairement acceptable de celui-ci pour une utilisation dans le traitement de la myiase d'un animal:

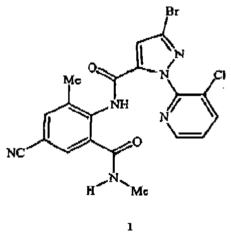

2. Composition selon la revendication 1, dans laquelle la myiase est entraînée au moins en partie par des larves choisies parmi les familles taxanomiques des Calliphoridés, des Sarcophagidés ou des Oestridés.

3. Composition selon la revendication 1, dans laquelle la myiase est entraînée au moins en partie par des larves de la famille des Calliphoridés.

4. Composition selon la revendication 1, dans laquelle la myiase est entraînée au moins en partie par des larves qui sont choisies dans le groupe constitué de *Lucilia cuprina* et *Lucilia sericata.*

5. Composition selon la revendication 1, dans laquelle la myiase est entraînée au moins en partie par des larves de *Lucilia cuprina.*

6. Composition selon la revendication 1, dans laquelle la myiase est entraînée au moins en partie par des larves de *Lucilia sericata.*

7. Composition selon la revendication 1 pour une utilisation dans le traitement de la myiase d'un animal selon la revendication 1, dans laquelle l'animal est un bovin ou un ovin.

8. Composition selon les revendications 1-7 pour une utilisation dans le traitement de la myiase d'un animal selon la revendication 1, où la composition comprend au moins un composant supplémentaire choisi dans le groupe constitué de solvants et/ou de supports, d'émulsifiants et/ou d'agents de dispersion.

9. Composition selon la revendication 8 pour une utilisation dans le traitement de la myiase d'un animal selon la revendication 1, où la composition comprend au moins un composé ou un agent biologiquement actif supplémentaire.

10. Composition selon la revendication 9 pour une utilisation dans le traitement de la myiase d'un animal selon la revendication 1, dans laquelle le composé ou l'agent biologiquement actif supplémentaire est choisi dans le groupe constitué de lactones macrocycliques, d'inhibiteurs d'acétyl-cholinestérase, de régulateurs de croissance d'arthropodes, d'antagonistes des canaux de chlorure à passage contrôlé par GABA, d'inhibiteurs de transport d'électrons mitochondriaux, d'agonistes/antagonistes/activateur d'acétylcholine nicotinique, d'inhibiteurs de phosphorylation oxydante, d'anthelminthiques, de modulateurs des canaux de sodium ou d'autres composés anti-parasitiques.

11. Composition selon la revendication 10 pour une utilisation dans le traitement de la myiase d'un animal selon la revendication 1, dans laquelle ledit composé biologiquement actif est une lactone macrocyclique.

12. Composition selon la revendication 10 pour une utilisation dans le traitement de la myiase d'un animal selon la revendication 1, dans laquelle ledit composé biologiquement actif est un inhibiteur d'acétyl-cholinestérase choisi dans le groupe d'organophosphates et d'organocarbamates.

13. Composition selon la revendication 10 pour une utilisation dans le traitement de la myiase d'un animal selon la revendication 1, dans laquelle ledit composé biologiquement actif est un régulateur de croissance d'arthropodes choisi dans le groupe d'inhibiteurs de synthèse de chitine, d'agonistes/perturbateurs d'ecdysone, d'inhibiteur de biosynthèse lipidique et de mimétiques d'hormones juvéniles.

14. Composition selon la revendication 10 pour une utilisation dans le traitement de la myiase d'un animal selon la revendication 1, dans laquelle ledit composé biologiquement actif est un antagoniste des canaux de chlorure à passage contrôlé par GABA.

15. Composition selon la revendication 10 pour une utilisation dans le traitement de la myiase d'un animal selon la revendication 1, dans laquelle ledit composé biologiquement actif est un inhibiteur de transport d'électrons mitochondriaux.

16. Composition selon la revendication 10 pour une utilisation dans le traitement de la myiase d'un animal selon la revendication 1, dans laquelle ledit composé biologiquement actif est un agoniste/antagoniste/activateur d'acétylcholine nicotinique.

17. Composition selon la revendication 10 pour une utilisation dans le traitement de la myiase d'un animal selon la revendication 1, dans laquelle ledit composé biologiquement actif est un inhibiteur de phosphorylation oxydante.

18. Composition selon la revendication 10 pour une utilisation dans le traitement de la myiase d'un animal selon la revendication 1, dans laquelle ledit composé biologiquement actif est un anthelminthique.

19. Composition selon la revendication 10 pour une utilisation dans le traitement de la myiase d'un animal selon la revendication 1, dans laquelle ledit composé biologiquement actif est un modulateur des canaux de sodium.
